## Europäisches Patentamt

## European Patent Office

## Office européen des brevets

(11) Publication number: **0 145 356**
**B1**

# EUROPEAN PATENT SPECIFICATION

(45) Date of publication of patent specification: **31.05.89**

(21) Application number: **84308022.7**

(22) Date of filing: **20.11.84**

(51) Int. Cl.⁴: **C 12 Q 1/68,** C 12 N 7/02, C 12 P 19/34

(54) Method of assay of DNA or RNA from virus particles.

(30) Priority: **22.11.83 GB 8331071**

(43) Date of publication of application:
**19.06.85 Bulletin 85/25**

(45) Publication of the grant of the patent:
**31.05.89 Bulletin 89/22**

(84) Designated Contracting States:
**CH DE FR GB IT LI NL**

(56) References cited:
**EP-A-0 079 139**
**WO-A-83/03678**

**GENE, vol. 21, 1983, pages 77-85, Elsevier Biomedical Press, Amsterdam, NL; M. RANKI et al.: "Sandwich hybridization as a convenient method for the detection of nucleic acids in crude samples"**

**JOURNAL OF MEDICAL VIROLOGY, vol. 9, 1982, pages 299-305, Alan R. Liss, Inc., US; D.D. RICHMAN et al.: "A rapid enzyme immunofiltration technique using monoclonal antibodies to serotype herpes simplex virus"**

(73) Proprietor: **NATIONAL RESEARCH DEVELOPMENT CORPORATION**
**101 Newington Causeway**
**London SE1 6BU (GB)**

(72) Inventor: **Karayiannis, Peter**
**3 Thurlestone Avenue**
**London N12 0LP (GB)**
Inventor: **Monjardino, Joao Pedro Pulido Valente**
**26 Daleham Gardens**
**London NW3 5DA (GB)**
Inventor: **Thomas, Howard Christopher**
**103 Oakwood Road**
**London NW11 6RJ (GB)**

(74) Representative: **Percy, Richard Keith**
**Patent Department National Research Development Corporation 101 Newington Causeway**
**London SE1 6BU (GB)**

Courier Press, Leamington Spa, England.

## EP 0 145 356 B1

(56) References cited:

GASTROENTEROLOGY, vol. 77, no. 5, 1979, page A46; J.R. WANDS et al.: "Production and characterization of monoclonal antibodies to hepatitis B surface antigen (HBsAg) by cellular hybridization"

NATURE, vol. 286, no. 5772, July 1980, pages 533-535, Basingstoke, GB; C. BRECHOT et al.: "Presence of integrated hepatitis B virus DNA sequences in cellular DNA of human hepatocellular carcinoma"

Infect Immunity (1980), vol. 28, 45-48
Hepatology (1987), vol. 1, 557-562
The Lancet (1976), pp. 1367-1371

## Description

### Background of the invention

#### 1. Field of the invention

This invention relates to a method of assay for viral DNA or RNA. The term "assay" is used herein to cover qualitative detection and quantitative or semi-quantitative measurement of the amount of DNA or RNA. The method is particularly applicable to the assay of a sample from a patient to determine whether he is suffering from infection by a particular kind of virus and, if so, how severe this infection is.

#### 2. Description of prior art

Hitherto viral particles have been extracted from a patient's serum by centrifuging. Viral DNA is extracted from the precipitate. Unfortunately, the precipitate includes a large amount of contaminating serum proteins. This has to be removed by adding protease and extracting the proteolysis products (mainly protein) into phenol. A large amount of protease is required and the extraction into phenol is an awkward operation from the standpoint of handling of the highly dangerous phenol. It is however necessary to remove protein fairly completely. If this is not done the viral DNA will not adhere to the nitrocellulose filter in the assay step which follows.

The sample DNA thus extracted is then assayable by the method of I. V. D. Weller *et al.*, J. Med. Virol. *9* 273—280 (1982). The DNA sample is put on to a nitrocellulose filter disc, danatured with alkali, neutralised and baked on to the nitrocellulose disc. The immobilised ss-DNA thus obtained is then hybridised to radio-labelled probe DNA and the radioactivity of the discs is then measured. A problem in this method is that the viral DNA present in the patient's serum must be free of protein to adhere to the nitrocellulose disc. Hence the need for removal of protein with phenol when using this technique.

### Summary of the invention

It has now been found that virus particles can be precipitated by use of an insolubilised monospecific, preferably monoclonal, antibody to a surface antigen of the virus and that the viral DNA or RNA can be extracted from this precipitate into the liquid phase without removing unwanted contaminating serum proteins which would be present in the pellet of virus obtained by centrifugation. All that is necessary is to add a small amount of some reagent, such as a protease, to release the viral DNA or RNA from any contaminating viral proteins, i.e. those closely associated with the viral DNA or RNA. The DNA or RNA is then made single-stranded, whereafter it is assayed by any method in which it is probed with an appropriate complementary DNA under hybridisation conditions and in which the presence of some protein in the extract is not detrimental. Since this method of extraction does not require the large amounts of protease previously used, it is cheaper and it is further highly advantageous in that it avoids the use of phenol, with all the safety problems involved in handling it.

The method of the invention is defined as a method of assay of DNA or RNA from virus particles present in a protein-containing clinical specimen which comprises the steps of

(a) precipitating virus particles from the specimen by incubating them with an insolubilised monospecific antibody to a viral surface antigen, thereby forming a precipitate comprising the virus particles and said antibody,

(b) removing the supernatant,

(c) breaking open the capsid of the virus particles of the precipitate to release the viral DNA or RNA thereof from any proteins associated therewith,

(d) extracting the viral DNA or RNA into the liquid phase,

(e) separating the liquid phase, which contains the DNA or RNA, from solid phase,

(f) where double-stranded DNA is being assayed, subjecting the extract thus obtained to conditions effective to render the DNA single-stranded, and

(g) assaying the DNA or RNA by a method in which it is probed with a labelled polynucleotide under hybridisation conditions and in which the presence of some protein in the extract is not detrimental.

It has further been found that the extract containing the single-stranded, viral DNA or RNA can be assayed efficiently and more accurately by a method in which two hybridisations are carried out, on different regions of the sample DNA or RNA, e.g. as described in EP—A—79139 or Gene *21*, 77—85 (1983). In the prior method of I. V. D. Weller *et al., loc. cit,* the sample is immobilised on the filter and a soluble, labelled probe DNA is employed, whereafter the unbound material is removed and the amount of label thereon determined. In the two-site method, the viral DNA or RNA is removed from the sample by hybridisation to part of the viral DNA (fragment A) attached to a solid phase. Simultaneously or separately a labelled probe DNA made from a different region of the viral DNA (fragment B) is hybridised to the sample viral DNA or RNA which has been, or will be, hybridised to the immobilised DNA. Thereby, if the sample contains the DNA of the virus under assay, it will under appropriate hybridisation conditions hybridise to both solid phase (fragment A) and labelled probe (fragment B). As a result, labelled insoluble material is produced. This is then separated from labelled soluble probe (fragment B), and subjected to an appropriate test for detecting or measuring the amount of label in the insoluble material.

Because the extracted sample viral DNA or RNA contains both viral and serum proteins, it is not appropriate to assay it by the prior art method in which the sample DNA or RNA is insolubilised on nitrocellulose by baking. The DNA or RNA would not stick properly to the nitrocellulose in the presence of protein. The extraction of protein would involve additional steps, use of phenol, and possible loss of nucleic acid, so it is much better not to treat the sample DNA or RNA further if it can be avoided. The two-site hybridisation assay therefore provides a way of avoiding further treatment of the sample DNA or RNA after extraction from the virus. It also improves the specificity of the assay. Although contaminating DNA from the serum might become trapped with the virus particles precipitated, just as in the prior method of centrifuging, the use of two separate hybridisations makes it less likely that false positives will be obtained.

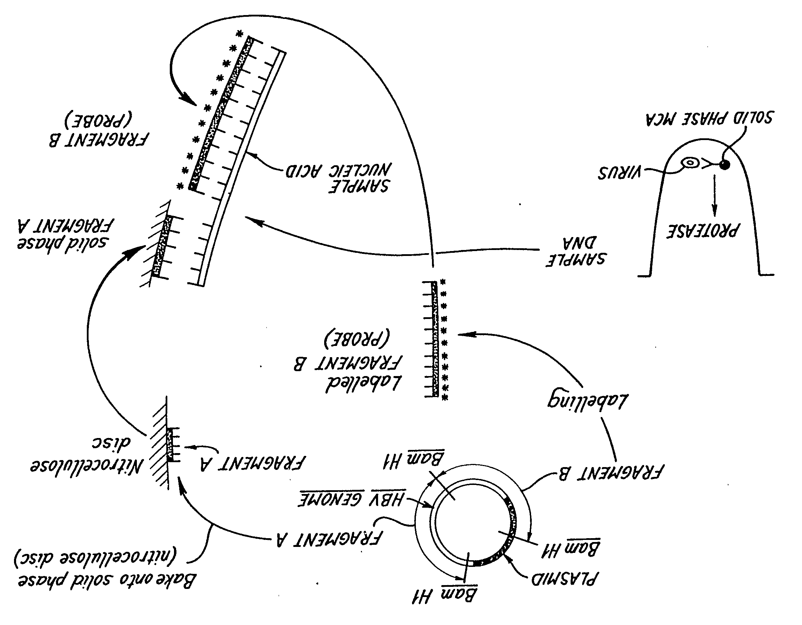

Brief description of the drawing

The accompanying drawing shows schematically the reagents and reactions involved in detection of hepatitis B virus (HBV).

Description of the preferred embodiments

The preferred two-site assay method can be defined as a method of assay of the liquid sample for DNA or RNA suspected of being present therein, which comprises subjecting the sample (the extract obtained as described) to conditions effective to render the DNA or RNA single-stranded, contacting the sample under hybridisation conditions with a first polynucleotide which is in a solid phase and unlabelled, and which is hybridisable under the assay conditions to a first region of the nucleic acid sequence of the suspected DNA or RNA and with a second polynucleotide which is in a liquid phase and labelled, thus serving as a probe, and which is hybridisable under the assay conditions to a second region, separate from the first region, of the nucleic acid sequence of the suspected DNA or RNA, separating the solid and liquid phases and determining the amount of label present in the solid phase. The first and second regions of the nucleic acid sequence must be mutually exclusive for hybridisation purposes, whereby the first polynuclotide cannot hybridise to the second region or the second polynucleotide to the first region.

The first polynucleotide, which is in the solid phase, need only have a short sequence length, from the minimum number of nucleotides needed to ensure an acceptable probability of unique hybridisation (which is usually taken to be in the range 14 to 17), upwards to any arbitrary limit governed by ease of hybridisation, e.g. 500. It can be made insoluble (insolubilised) by baking onto nitrocellulose discs or by chemical linkage to an insolubilised support of some other kind or by a method in which the support is provided with a DNA sequence by which it is attached to a complementary DNA sequence on the DNA of the first polynucleotide. The last-mentioned method implies, of course, that the first polynucleotide has a DNA sequence long enough to provide two hybridisation sites, one for attachment to the sample DNA and the second for attachment to the support DNA. In other words, the overall assay would involve three hybridisations. An advantage of this embodiment is that it lends itself to the "dipstick" technique, whereas baking onto a support does not. For the dipstick technique, the support has a DNA "arm", one end of which is baked onto the solid material and the other end of which contains the DNA sequence complementary to the first region of the sample DNA. The support is then lowered into the solution under assay and incubated to effect the hybridisation.

The second polynucleotide, the probe, which is in solution, is labelled and will usually have a longer sequence than the first, although not necessarily so. A typical length is from 50 to 2000 nucleotides. It can be labelled by conventional radiolabelling, e.g. with $^{32}$P. It can alternatively be enzyme-labelled, a technique for doing this having been described by A. D. B, Malcolm *et al.*, on 1 July 1983 at the 604th Biochemical Society meeting, Cambridge, England, using horseradish peroxidase enzyme. Abstracts of this meeting have been published and the disclosure of the relevant abstract is herein incorporated by reference. Other kinds of label can be used. Thus the biotin labelling method of D. C. Ward and his colleagues at Yale University, described in European Patent Specification 63879, can be used. In this method biotin groups are attached chemically to deoxyribonucleotides, especially biotin-11-dUTP, and introduced into DNA by nick-translation. From this biotin-labelled ds-DNA, they prepared single-stranded DNA having a sequence complementary to a DNA sequence to be detected, and the resultant biotin-labelled probe DNA is contacted under hybridisation conditions with the DNA sequence to be detected suitably immobilised e.g. on a nitrocellulose filter. To detect hybridisation, use is made of a biotin/streptavidin, biotin/avidin, biotin/anti-biotin or similar interaction. The streptavidin etc. is then labelled, e.g. by fluorescence or enzymatically, using e.g. biotin-conjugated alkaline phosphatase.

Both polynucleotides are conveniently fragments, shown in the drawing as fragments A and B, prepared from the viral genome, conveniently by recombinant DNA technology. Thus, the drawing shows fragments obtained by enzymic digest of cHBV-DNA with *Bam* H1. The preparation of recombinant DNA is now conventional technology. In the case of hepatitis B virus it has been found possible to use the whole plasmid containing an insert of the whole length of the viral DNA. By using *Bam* H1 three cuts are made, two within the viral DNA and a third within the plasmid DNA, whereby one fragment consists of DNA alone and the other of viral DNA tailed with plasmid DNA.

The two polynucleotides can consist entirely of single-stranded (ss) DNA or can be partly double-stranded while having single-stranded portions effective for the intended hybridisations. The term "single-stranded" or "hybridisable" should be understood to imply the presence of the relevant sequence in ss-form, and references herein to making the polynucleotide single-stranded should be construed as making at least the relevant sequence thereof single-stranded. Methods of making ss-DNA from ds-DNA including use of strong alkali or heat, for example, are well known in themselves.

Normally the same hybridisation conditions can be used for each polynucleotide. Thus, conveniently the two polynucleotides are contacted substantially simultaneously with the sample. That is, they can be added precisely simultaneously or in either order, one immediately after the other, to the sample.

The amount of label in the insoluble material after the hybridisations can be measured by any method known from conventional antigen/antibody immunoassay technology and dependent on the kind of label used. Thus the label can be measured directly from the insoluble material or it can be calculated by measurements made in the liquid phase and compared with standards.

All viruses have surface antigens. Generally stated, all viruses have a nucleus containing DNA or RNA and a capsid or coat surrounding the nucleus and containing translated protein. Some viruses have an envelope covering the capsid which usually consists of lipids, proteins and carbohydrates. Others do not have this envelope and are described as naked. The enveloped viruses have surface antigens located within the envelope. The naked viruses have surface antigens on the capsid surface. Examples of viruses to which the invention is applicable are viruses of the hepatitis B family, hepatitis D, herpetoviridae, especially herpes zoster, cytomegalovirus (CMV), herpes simplex, EB virus, poxviridae, especially smallpox, vaccinia, parvoviridae, especially the adenoviruses, the papavoviridae, picornaviridae, e.g. poliomyelitis, FMD virus and hepatitis A, reoviridae, rotaviruses, paramyxoviridae, especially measles, distemper and respiratory synsitial viruses, orthomyxoviridae, especially influenza viruses, rhabdoviridae, especially rabies viruses, togaviridae, especially the arboviruses, rubella and yellow fever, and the coronaviridae, Marburg and Edola viruses. Hepatitis B is, of course, associated with liver damage. Cytomegalovirus (CMV) is responsible for severe damage to many organs in newly born babies and is transmissible from the mother during pregnancy.

The term "monospecific antibody" as used herein includes monoclonal and monospecific polyvalent antibodies, the latter being polyvalent for different sites on the same virus.

The method of the invention depends for its effectiveness on use of an antibody to precipitate the virus particles highly selectively. Methods of making monoclonal antibodies (MCAs) are now well known and many are commercially available. See e.g. EPC Application 38642 (NRDC) for monoclonal antibodies to hepatitis B. The surface antigen provides a convenient point at which to attach the MCA and thereby form an antibody-antigen complex. The antibody is pre-insolubilised, e.g. by linking it to an agarose gel in a manner well known

*per se* and the virus particle is thus attracted to the support. Less insoluble material including substantially all the serum protein is then easily removed by relatively low-speed centrifugation. Capsid protein remains within the virus particle. However, it is not necessary to extract it to release the viral DNA or RNA. The capsid can be broken open very easily and the DNA or RNA released from the associated proteins by a small amount of enzyme, far less than is required for proteolysis of all the serum protein encountered in the above-described prior method. Generally, less than 50% of the enzyme previously required, preferably less than 35% is used. Following this step, the DNA or RNA enters an aqueous phase very easily, enabling ample quantities to be extracted for assay. The aqueous phase inevitably contains some capsid protein, but this is unimportant, since the two-site assay method of the invention is unaffected by the presence of protein.

The invention is applicable to all kinds of virus, especially those infective in humans, non-human animals, or plants. The samples can be derived from patients suspected of infection by the virus and can be serum or urine samples, for example. The sample can also be of blood in a blood bank, to check its purity, for example. Some viruses have RNA in their nucleus but can be assayed in similar fashion using *c*DNA polynucleotides.

The invention includes specifically a kit for use in the method of assay of viral DNA or RNA, involving the combination of extraction of viral DNA and RNA and two-site hybridisation assay, comprising:

(1) a monospecific, preferably monoclonal, antibody to the surface antigen of a virus;

(2) a first polynucleotide which is unlabelled and which, when in single-stranded form is hybridisable to a first region of the nucleic acid sequence of the viral DNA or RNA; and

(3) a second polynucleotide which is labelled and which, when in single-stranded form, is hybridisable to a second region of the nucleic acid sequence of the viral DNA or RNA, separate from the first.

The kit can also contain any one or more of the following:

(4) a support material for insolubilising the monospecific antibody thereon;

(5) a support material for insolubilising the first polynucleotide in ss-form;

(6) a "control" DNA or RNA for testing the kit, the control DNA or RNA comprising a sequence which, when the control DNA or RNA is in single-stranded form, is hybridisable, to each polynucleotide, when the polynucleotides are in single-stranded form; and

(7) a reagent, especially an enzyme, for breaking open the capsid of the virus and for releasing the viral DNA or RNA from proteins closely associated therewith.

It will be appreciated that the polynucleotides and the control DNA or RNA supplied in such kits can be in ds-form and can be rendered single-stranded *in situ* by the buyer of the kit.

The following Example illustrates the invention.

Example 1
Hepatitis B virus
(1) Preparation of viral DNA from hepatitis B virus

CNBr-activated "Sepharose" 4B® coated with RFHBs-1 (monoclonal antibody to the "a" determinant of hepatitis B surface antigen, 10 mg protein/ml of beads) was used to sediment virus particles from patients' sera. 50 microlitres of packed "Sepharose"® beads coated as above were therefore mixed with 150 microlitres of patient's serum and incubated at room temperature for 2 hours. The beads were allowed to settle and the supernatant was removed and replaced with 150 microlitres of a mixture containng (20 mM Tris-HCl pH 8, 150 mM NaCl, 10 mM EDTA, 0.2% SDS and 1 mg/ml Pronase). The samples were incubated at 37°C for 2 hours and the supernatant containing the viral DNA was removed.

(2) Preparation of the first polynucleotide, in the solid phase (fragment A)

Viral DNA purified from the hepatitis B virus DNA plasmid pBH20-HBV was cut in two by digestion with *Bam* H1. The two fragments were separated by preparative agarose electrophoresis. The longer fragments (B) were used as described in the next section. The shorter fragments (A) were made single-stranded by treatment with NaOH and neutralised with Tris-HCl pH 7.0 and were then immobilised on nitrocellulose discs. These were baked at 80°C for 2 hours.

(3) Preparation of the second polynucleotide, the labelled probe (fragment B)

The longer fragments (B) of the *Bam* H1-digested pBH20-HBV plasmid were nick-translated uniformly with $^{32}P$ to high specific activity using radiolabelled nucleotides.

(4) Assay of the viral DNA

40 microlitres of the extracted viral DNA were denatured with 10 microlitres of alkali for 5 minutes and the neutralised with 10 microlitres of Tris-HCl pH 7.0. 20 ng/ml of the above HBV $^{32}P$-DNA were added to the hybridisation mix. This was heated in a boiling water bath for 3 minutes and then cooled on ice. 140 microlitres of this mix was added to each denatured test sample so that the final concentration of the constituent components of the hybridisation mix were 5×SSC (0.015 M sodium citrate, 0.15 M NaCl), 0.5 mg/ml each Ficol®, BSA, PVP and SDS, and 50% v/v formamide. The sample-probe mixtures were placed in 10 mm diameter wells in polyvinyl trays and a nitrocellulose disc, prepared as in (2) above, was dropped into each well. Hybridisation was allowed to proceed for 15 hours at 37°C. The discs were then washed and counted in a scintillation counter (as described by I. V. D. Weller *et al., loc. cit.*).

Example 2
Cytomegalovirus
(1) Preparation of viral DnA from cytomegalovirus

Example 1, Stage (1) can be repeated except that the beads are coated with cytomegalovirus neutralising monoclonal antibodies [L. Pereira *et al.,* Infect. Immun. 36; 924—932 (1982)]. 10 mg protein/ml of beads can be used to sediment virus from patients' urine following incubation at room temperature for 2 hours. Immobilised virus on the solid phase can be digested with 150 microlitres of 20 mM tris HCl pH 8.0, 150 mM NaCl, 10 mM EDTA, 0.2% SDS and 1 mg/ml pronase. After incubation at 37°C for 2 hours the supernatant containing the viral DNA can be removed.

(2) and (3) Preparation of DNA fragments (first and second polynucleotides)

Suitable cytomegalovirus DNA fragments selected from the viral sequences cloned in recombinant plasmids in *E. coli* [J. D. Oram *et al.,* J. Gen. Virol., *59;* 111—129 (1982)] can be used in probing viral extracts. This large viral genome has several *Eco*RI, *Bam*HI, *Hind*III etc., restriction sites. It can therefore be treated with a restriction endonuclease to yield several fragments. Two such fragments having no homology between them can be used for hybridisation. One particularly convenient procedure is to use the fragment $H_{12}B_2$ of Oram *et al.,* which happens to have only one *Bgl*II restriction site. This is cut into a small fragment (A) and a larger one (B). One (A) is denatured with NaOH, neutralised with Tris-HCl pH 7.0, and immobilised on nitrocelluose discs, followed by baking at 80°C for 2 hours. The other fragment (B) is nick-translated with $^{32}P$ to high specific activity using radiolabelled nucleotides.

Example 3
Hepatitis D virus
(1) Preparation of the viral DNA

The coat of the hepatitis D virus ($\frac{3}{4}$-agent) is composed of hepatitis B surface antigen and the nucleocapsid of $\frac{3}{4}$ antigen. Virus particles can be precipitated either by using CNBr-activated 'Sepharose' (4B® coated with RF-HBs-1 (10 mg protein/ml of beads), monoclonal antibody to hepatitis B surface antigen or, following treatment with detergent (0.3% NP40) by a solid phase, coated with polyclonal anti-$\frac{3}{4}$-agent (10 mg protein/ml of beads). Digestion with 150 microlitres of 20 mM Tris-HCl pH 8.0, 15 mM NaCl, 10 mM EDTA, 0.2% SDS and 1 mg/ml pronase of the immobilised virus particles or nucleocapsids release the RNA nucleic acid which can then be probed with fragments from cDNA copies of the RNA genome.

(2) and (3) Preparation of cDNA fragments

First, the genome RNA is poly-A tailed at its 3'-end in conventional manner. The RNA is reverse transcribed to produce cDNA of various lengths up to a molecular weight of $5\times10^5$. The cDNA is inserted into plasmid pBR 322. Amplified cDNA copies can be excised and treated with restriction endonuclease to yield fragments which are treated as in Example 2, Stages (2) and (3).

(4) Assay of the viral RNA

The same procedure is followed as already described, but unlike hepatitis B virus or cytomegalovirus, the alkali step treatment which destroys RNA is omitted. The samples can be placed directly into the hybridisation mix.

**Claims**

1. A method of assay of DNA or RNA from virus particles present in a protein-containing clinical specimen which comprises the steps of

(a) precipitating virus particles from the specimen by incubating them with an insolubilised monospecific antibody to a viral surface antigen, thereby forming a precipitate comprising the virus particles and said antibody,

(b) removing the supernatant,

(c) breaking open the capsid of the virus particles of the precipitate to release the viral DNA or RNA thereof from any proteins associated therewith,

(d) extracting the viral DNA or RNA into the liquid phase,

(e) separating the liquid phase, which contains the DNA or RNA, from solid phase,

(f) where double-stranded DNA is being assayed, subjecting the extract thus obtained to conditions effective to render the DNA single-stranded, and

(g) assaying the DNA or RNA by a method in which it is probed with a labelled polynucleotide under hybridisation conditions and in which the presence of some protein in the extract is not detrimental.

2. A method according to Claim 1 wherein the capsid is broken open using a protease enzyme.

3. A method according to Claim 2 wherein the capsid is reacted with a protease in a concentration lower than that which would be required for proteolysis of serum proteins present in the specimen.

4. A method according to Claim 1, 2 or 3 wherein the monospecific antibody is a monoclonal antibody.

5. A method according to any preceding Claim wherein the virus is a hepatitis B, hepatitis D or herpetoviridae virus.

6. A method according to any preceding Claim wherein in step (g) said probing comprises contacting the DNA or RNA under hybridisation conditions with a first polynucleotide which is in a solid phase and unlabelled, and which is hybridisable under the assay conditions to a first region of the nucleic acid sequence of the suspected DNA or RNA and with a second polynucleotide which is in a liquid phase and labelled and which is hybridisable under the assay conditions to a second region of the nucleic acid sequence of the suspected DNA or RNA, the second region being separate from the first and mutually exclusive therefrom with respect to hybridisation, separating the solid and liquid phases and determining the amount of label present in the solid phase.

7. A method according to Claim 6 wherein the hybridization conditions are the same for the first and second polynucleotides and they are contacted substantially simultaneously with the sample.

8. A kit for use in assaying DNA or RNA from virus particles present in a protein-containing clinical specimen, said kit comprising in separate containers:

(a) a monospecific antibody to the surface antigen of a virus;

(b) a first polynucleotide which is unlabelled and which, when in single-stranded form, is hybridisable to a first region of the nucleic acid sequence of the viral DNA or RNA; and

(c) a second polynucleotide which is labelled and which, when in single-stranded form, is hybridisable to a second region of the nucleic acid sequence of the viral DNA or RNA, separate from the first and mutually exclusive therefrom with respect to hybridisation.

9. A kit according to Claim 8 which further comprises at least one additional component selected from the group consisting of:

(a) a support material for insolubilising the specific antibody thereon;

(b) a support material for insolubilising the first polynucleotide in single-stranded form;

(c) a control DNA or RNA for testing the kit, the control DNA or RNA comprising a sequence which, when the control DNA or RNA is in single-stranded form, is hybridisable to each polynucleotide, when the polynucleotides are in single-stranded form; and

(d) a reagent for breaking open the capsid of the virus to release the viral DNA or RNA from any proteins associated therewith, each such additional component being present in a separate container.

10. A kit according to Claim 9 wherein the reagent for breaking open the capsid of the virus is a protease enzyme.

11. A kit according to Claim 8, 9 or 10 wherein the monospecific antibody is a monoclonal antibody.

12. A kit according to Claim 8, 9, 10 or 11 wherein the virus is a hepatitis B, hepatitis D, or herpetoviridae virus.

**Patentansprüche**

1. Verfahren zum Testen von DNS oder RNS aus Virusteilchen, die in einer proteinhaltigen klinischen Probe vorliegen, gekennzeichnet durch die folgenden Stufen:

a) Ausfällung der Virusteilchen aus der Probe durch Inkubieren derselben mit einem in feste Form gebrachten monospezifischen Antikörper gegen ein virales Oberflächenantigen und dadurch Bildung eines Niederschlages, der die Virusteilchen und diesen Antikörper enthält,

b) Entfernung des Überstandes,

c) Aufbrechen des Capsids der Virusteilchen des Niederschlages zur Freisetzung der viralen DNS oder RNS von allen ihn begleitenden Proteinen,

d) Extraktion der viralen DNS oder RNS in die flüssige Phase,

e) Abtrennung der flüssigen Phase, welche die DNS oder RNS enthält von der festen Phase,

f) beim Testen von doppelsträngiger DNS wird der so erhaltene Extrakt Bedingungen unterworfen, welche die DNS einzelsträngig machen, und

g) Testen der DNS oder RNS nach einer Methode, bei welcher sie einem Test mit einem markierten Polynukleotid als Sonde unter Hybridisierungsbedingungen unterworfen wird, und bei welcher das Vorliegen von etwas Protein im Extrakt nicht schädlich ist.

2. Verfahren nach Anspruch 1, dadurch gekennzeichnet, daß das Capsid unter Verwendung einer Protease aufgebrochen wird.

3. Verfahren nach Anspruch 2, dadurch gekennzeichnet, daß das Capsid mit einer Protease in einer geringeren Konzentrations als derjenigen, die zur Proteolyse der in der Probe vorhandenen Serumproteine nötig wäre, umgesetzt wird.

4. Verfahren nach Anspruch 1 bis 2 oder 3, dadurch gekennzeichnet, daß der monospezifische Antikörper ein monoklonaler Antikörper ist.

5. Verfahren nach einem der vorhergehenden Ansprüche, dadurch gekennzeichnet, daß das Virus eine Hepatitis B-, Hepatitis D- oder Herpetoviridae-Virus ist.

6. Verfahren nach einem der vorhergehenden Ansprüche, dadurch gekennzeichnet, daß in Stufe (g) der Test mit der Sonde dadurch geschieht, daß die DNS oder RNS unter Hybridisierungsbedingungen mit einem ersten Polynukleotid, das in fester Phase vorliegt und nicht markiert ist, und das unter den Testbedingungen mit einer ersten Region der Nukleinsäuresequenz der vermuteten DNS oder RNS hybridisierbar ist, danach mit einem zweiten Polynukleotid, das in flüssiger Phase vorliegt und markiert ist, und das unter den Testbedingungen zu einer zweiten Region der Nukleinsäuresequenz der vermuteten DNS oder RNS hybridisierbar ist, in Kontakt gebracht wird, wobei die zweite Region von der ersten getrennt ist und beide nicht miteinander kreuzhybridisierbar sind, und schließlich feste und die flüssige Phase getrennt werden und die Menge an in der festen Phase vorhandener Markierung bestimmt wird.

7. Verfahren nach Anspruch 6, dadurch gekennzeichnet, daß die Hybridisierungsbedingungen für das erste und das zweite Polynukleotid gleich sind und daß beide praktisch gleichzeitig mit der Probe in Kontakt gebracht werden.

8. Kit zur Verwendung beim Testen von DNS oder RNS aus Virusteilchen, die in einer proteinhaltigen klinischen Probe vorliegen, dadurch gekennzeichnet, daß dieses Kit in getrennten Behältern enthält:

a) einen monospezifischen Antikörper gegen ein Oberflächenantigen eines Virus;

b) ein erstes Polynukleotid, das nicht markiert ist und das in einzelsträniger Form zu einer ersten Region der Nukleinsäuresequenz der viralen DNS oder RNS hybridisierbar ist und

c) ein zweites Polynukleotid, das markiert ist und das, in einzelsträniger Form, zu einer zweiten Region der Nukleinsäuresequenz der viralen DNS oder RNS hybridisierbar ist, die von der ersten getrennt und mit ihr nicht kreuzhybridisiert.

9. Kit nach Anspruch 8, dadurch gekennzeichnet, daß es weiter zumindest einer zusätzliche Komponente aus der folgenden Gruppe enthält:

a) ein Trägermaterial, um den spezifischen Antikörper darauf in feste Form zu bringen;

b) ein Trägermaterial, um das erste Polynukleotid in einzelsträniger Form in feste Form zu bringen;

c) eine Kontroll-DNS oder -RNS zum Testen des Kits, wobei die Kontroll-DNS oder -RNS eine Sequenz enthält, die, wenn die Kontroll-DNS oder -RNS in einzelsträniger Form vorliegt, zu jedem der beiden Polynukleotide hybridisierbar ist, wenn diese in einzelsträniger Form vorliegen, und

d) ein Reagenz zum Aufbrechen des Capsids des Virus zur Freisetzung der viralen DNS oder RNS von allen begleitenden Proteinen, wobei jede solche zusätzliche Komponente in einem getrennten Behälter vorliegt.

10. Kit nach Anspruch 9, dadurch gekennzeichnet, daß das Reagenz zum Aufbrechen des Capsids des Virus eine Protease ist.

11. Kit nach Anspruch 8, 9 oder 10, dadurch gekennzeichnet, daß der monospezifische Antikörper ein monoklonaler Antikorper ist.

12. Kit nach Anspruch 8, 9, 10 oder 11, dadurch gekennzeichnet, daß das Virus ein Hepatitis B-, Hepatitis D- oder Herpetoviridae-Virus ist.

**Revendications**

1. Procédé de dosage de l'acide désoxyribonucléique (ADN) ou de l'acide ribonucléique (ARN) provenant de particules virales présentes dans un échantillon clinique contenant de la protéine, ce procédé comprenant les étapes consistant à:

(a) précipiter les particules virales de l'échantillon, en les faisant incuber avec un anticorps, insolubilisé, monospécifique d'un antigène de la surface virale, en formant ainsi un précipité comprenant les particules virales et ledit anticorps,

(b) enlever le liquide surnageant,

(c) ouvrir, par rupture, la capside des particules virales du précipité afin de dégager leur ADN ou ARN et de les séparer de toutes les protéines éventuellement associées à elle,

(d) extraire l'ADN ou l'ARN viral pour le faire passer dans la phase liquide,

(e) séparer la phase liquide, qui contient l'ADN ou l'ARN, de la phase solide,

(f) lorsque de l'ADN en double hélice est soumis à dosage, soumettre l'extrait ainsi obtenu à des conditions capables efficacement de rendre monocaténaire l'ADN, et

(g) doser l'ADN ou l'ARN par un procédé dans lequel on le soumet à essai avec un polynucléotide marqué, dans des conditions d'hybridation, et dans lequel la présence d'un peu de protéine dans l'extrait n'est pas nuisible.

2. Procédé selon la revendication 1, dans lequel la capside est ouverte, avec rupture, grâce à l'utilisation d'une enzyme de type protéase.

3. Procédé selon la revendication 2, dans lequel on fait réagir la capside avec une protéase en une concentration inférieure à celle qui serait nécessaire pour réaliser la protéolyse des protéines de sérum présentes dans l'échantillon.

4. Procédé selon la revendication 1, 2 ou 3, dans lequel l'anticorps monospécifique est un anticorps monoclonal.

5. Procédé selon l'une quelconque des revendications précédentes, dans lequel le virus est un virus de l'hépatite B, de l'hépatite D ou du type herpes-virus.

6. Procédé selon l'une quelconque des revendications précédentes, dans lequel, à l'étape (g) l'essai consiste à mettre l'ADN ou l'ARN, dans des conditions d'hybridation, en contact avec un premier polynucléotide qui est en phase solide et n'est par marqué, et qui, dans les conditions de dosage, peut subir une formation d'hybrides par fixation sur une première région de la séquence des acides nucléiques de l'ADN ou l'ARN soupçonnés, et avec un second polynuclétide qui est en phase liquide et est marqué, et qui, dans les conditions de dosage, peut se fixer par hybridation sur une second région de la séquence des acides nucléiques de l'ADN ou de l'ARN soupçonnés, la seconde région étant séparée de la première région et les deux étant mutuellement exclusives en ce qui concerne une hybridation, puis la séparation des phases solide et liquide et la détermination de la quantité de marqueur présente en phase solide.

7. Procédé selon la revendication 6, dans lequel les conditions d'hybridation sont les mêmes pour le premier polynucléotide et le second polynucléotide, et on les met en contact de façon sensiblement simultanée avec l'échantillon.

8. Nécessaire destiné à servir à doser l'ADN ou l'ARN de particules virales présentes dans un échantillon clinique contenant des protéines, ledit nécessaire contenant, dans des récipients séparés:

(a) un anticorps monospécifique de l'antigène de surface d'un virus;

(b) un premier polynucléotide, qui n'est pas marqué et qui, lorsqu'il est sous forme monocaténaire, peut se fixer par hybridation sur une première région de la séquence des acides nucléiques de l'ADN ou de l'ARN viral; et

(c) un second polynucléotide qui est marqué et qui, quand il est sous forme monocaténaire, peut se fixer par hybridation sur une seconde région de la séquence des acides nucléiques de l'ADN ou de l'ARN viral, séparée de la première et en étant mutuellement exclusive en ce qui concerne l'hybridation.

9. Nécessaire selon la revendication 8, qui comprend en outre au moins un composant supplémentaire choisi parmi:

(a) une matière de support pour y fixer, par insolubilisation, l'anticorps spécifique;

(b) une matière de support pour fixer par insolubilisation le premier nucléotide sous forme monocaténaire;

(c) un ADN ou ARN témoin pour étalonner ou vérifier le nécessaire, l'ADN ou ARN témoin comprenant une séquence qui, lorsque l'ADN ou l'ARN témoin est sous forme monocaténaire, peut se fixer par hybridation sur chaque polynucléotide, lorsque les polynucléotides sont sous forme monocaténaire; et

(d) un réactif pour ouvrir par rupture la capside du virus afin de dégager l'ADN ou l'ARN viral et le séparer de toutes les protéines éventuellement associées à l'acide, chaque composant supplémentaire de ce genre étant présent dans un récipient séparé.

10. Nécessaire selon la revendication 9, dans lequel le réactif destiné à ouvrir par rupture la capside du virus est une enzyme de type protéase.

11. Nécessaire selon la revendication 8, 9 ou 10, dans lequel l'anticorps monospécifique est un anticorps monoclonal.

12. Nécessaire selon la revendication 8, 9, 10 ou 11, dans lequel le virus est un virus de l'hépatite B, de l'hépatite D ou du type herpes-virus.

Bake onto solid phase
(nitrocellulose disc)

Nitrocellulose disc

solid phase FRAGMENT A

FRAGMENT B (PROBE)

FRAGMENT A

FRAGMENT A

HBV GENOME

Bam H1

Bam H1

Bam H1

Bam H1

PLASMID

FRAGMENT B

Labelled FRAGMENT B (PROBE)

Labelling

SAMPLE NUCLEIC ACID

SAMPLE DNA

VIRUS

PROTEASE

SOLID PHASE MCA